# EUROPEAN PATENT SPECIFICATION

(11) **EP 0 696 194 B1**
(45) Date of publication and mention of the grant of the patent: **21.08.2002**
(21) Application number: 94912048.9
(22) Date of filing: 26.04.1994
(51) Int. Cl.: A61K 31/40, A61P 9/12, A61P 25/06, A61P 25/24

(54) **USE OF INDOLE DERIVATIVES AS 5HT1 ANTAGONISTS**
VERWENDUNG VON INDOLDERIVATEN ALS 5HT1-AGONISTEN
UTILISATION DE DERIVES D'INDOLES COMME ANTAGONISTES DE 5HT1

(30) Priority: 27.04.1993 US 53930
(43) Date of publication of application: 14.02.1996
(73) Proprietor: PFIZER INC., New York, N.Y. 10017 (US)
(72) Inventor: MACOR, John Eugene, Salem, CT 06420 (US)
(74) Representative: Wood, David John
(86) International application number: IB9400079
(87) International publication number: WO94025023

(56) References cited:
- WO-A-92/06973
- BRAIN RESEARCH, vol.626, no.1-2, 29 October 1993 pages 303 - 304 LEE, W.S. ET AL 'CONFORMATIONALLY RESTRICTED SUMATRIPTAN ANALOGUES, CP-122,288 AND CP-122,638 EXHIBIT ENHANCED POTENCY AGAINST NEUROGENIC INFLAMMATION IN DURA MATER' cited in the application

## Description

### Background of the Invention

The present invention relates to pharmaceutical compositions containing indole derivatives and to their medicinal use. The active compounds of the present invention are useful in treating migraine and other disorders.

United States Patents 4,839,377 and 4,855,314 and European Patent Application Publication Number 313397 refer to 5-substituted 3-aminoalkyl indoles. The compounds are said to be useful for the treatment of migraine.

British Patent Application 040279 refers to 3-aminoalkyl-1H-indole-5-thioamides and carboxamides. The compounds are said to be useful in treating hypertension, Raymond's disease and migraine.

European Patent Application Publication Number 303506 refers to 3-poly:hydro-pyridyl-5-substituted-1H-indoles. The compounds are said to have 5HT₁-receptor agonist and vasoconstrictor activity and to be useful in treating migraine.

European Patent Application Publication Number 354777 refers to N-piperidinyl:indolyl:ethyl-alkane sulfonamide derivatives. The compounds are said to have 5HT₁-receptor agonist and vasoconstrictor activity and to be useful in treating cephalic pain.

The compounds are generically disclosed in International Publication. No. WO 92/06973.

### Summary of the Invention

The present invention relates to pharmaceutical compositions and methods of use of (R)-5-(methylaminosulfonylmethyl)-3-(N-methylpyrrolidin-2-ylmethyl)-1H-indole and (R)-5-(methylaminosulfonylmethyl)-3-(pyrrolidin-2-ylmethyl)-lH-indole (hereinafter also referred to as the active indoles).

The present invention relates to a pharmaceutical composition for treating a condition selected from hypertension, depression, anxiety, eating disorders, obesity, drug abuse, cluster headache, migraine, pain, and chronic paroxysmal hemicrania and headache associated with vascular disorders comprising an amount of a compound of the active indoles or a pharmaceutically acceptable salt thereof effective in treating such condition and a pharmaceutically acceptable carrier.

The present invention also relates to a pharmaceutical composition for treating disorders arising from deficient serotonergic neurotransmission (e.g., depression, anxiety, eating disorders, obesity, drug abuse, cluster headache, migraine, pain, and chronic paroxysmal hemicrania and headache associated with vascular disorders) comprising an amount of a compound of the active indoles or a pharmaceutically acceptable salt thereof effective in treating such condition and a pharmaceutically acceptable carrier.

The present invention also relates to a method for treating a condition selected from hypertension, depression, anxiety, eating disorders, obesity, drug abuse, cluster headache, migraine, pain and chronic paroxysmal hemicrania and headache associated with vascular disorders.

The present invention also relates to a method for treating disorders arising from deficient serotonergic neurotransmission.

### Detailed Description of the Invention

The active indoles used in the present invention can be prepared using the methods disclosed in International Publication No. WO 92/06973.

The active indoles are capable of forming a wide variety of different salts with various inorganic and organic acids. Although such salts must be pharmaceutically acceptable for administration to animals, it is often desirable in practice to initially isolate an active indoles from the reaction mixture as a pharmaceutically unacceptable salt and then simply convert the latter back to the free base compound by treatment with an alkaline reagent, and subsequently convert the free base to a pharmaceutically acceptable acid addition salt. The acid addition salts of the active indoles are readily prepared by treating the compound with a substantially equivalent amount of the chosen mineral or organic acid in an aqueous solvent medium or in a suitable organic solvent such as methanol or ethanol. Upon careful evaporation of the solvent, the desired solid salt is obtained.

The acids which are used to prepare the pharmaceutically acceptable acid addition salts of the active indoles are those which form non-toxic acid addition salts, i.e., salts containing pharmacologically acceptable anions, such as hydrochloride, hydrobromide, hydroiodide, nitrate, sulfate or bisulfate, phosphate or acid phosphate, acetate, lactate, citrate or acid citrate, tartrate or bitartrate, succinate, maleate, fumarate, gluconate, saccharate, benzoate, methanesulfonate and pamoate [i.e., 1,1'-methylene-bis-(2-hydroxy-3-naphthoate)] salts.

The active indoles and the pharmaceutically acceptable salts thereof (hereinafter, also referred to as the active compounds) are useful psychotherapeutics and are potent serotonin (5-HT₁) agonists and may be used in the treatment of depression, anxiety, eating disorders, obesity, drug abuse, cluster headache, migraine, chronic paroxysmal hemicrania and headache associated with vascular disorders, pain, and other disorders arising from deficient serotonergic neurotransmission. The compounds can also be used as centrally acting antihypertensives and vasodilators.

The active compounds of the invention are evaluated as anti-migraine agents by testing the extent to which they mimic sumatriptan in contracting the dog isolated saphenous vein strip (P.P.A. Humphrey et al., Br. J. Pharmacol., 94, 1128 (1988)). This effect can be blocked by methiothepin, a known serotonin antagonist. Sumatriptan is known to be useful in the treatment of migraine and produces a selective increase in carotid vascular resistance in the anaesthetized dog. It has been suggested (W. Fenwick et al., Br. J. Pharmacol., 96, 83 (1989)) that this is the basis of its efficacy.

The active compounds of the present invention are also evaluated as anti-migraine agents via the inhibition of plasma protein extravasation response within the dura mater of guinea pigs following unilateral electrical trigeminal ganglion stimulation. The extent to which they mimic sumatriptan, in terms of both potency and efficacy, is determined in this assay. The procedure is performed on male Hartley guinea pigs (200-250 g, Charles River Laboratories, Wilmington, MA, U.S.A.) as described in Markowitz et al., J. Neurosci., 7 (12), 4129-4136 (1987) and also in Lee, et al., Brain Reseach, 626, 303-305 (1993). The procedure briefly consists of placing pentobarbitone-anesthetized animals in a stereotaxic frame. ¹²⁵I-BSA (bovine serum albumin) (50 µCi/kg⁻¹) is first injected into the femoral vein, followed 5 minutes later by drug or vehicle. Bipolar electrodes are then lowered into the trigeminal ganglia, and the right ganglion is stimulated for 5 minutes (1.2 nA, 5 Hz, 5 msec). The animal is then perfused with saline through the left cardiac ventricle and sacrificed, and the dura mater is dissected, weighed, and counted for radioactivity. Cpm/mg wet weight values are determined for the right vs left dura mater, and a ratio for the stimulated vs unstimulated sides is generated for each animal. Unpaired student's t-test is used to statistically compare these ratio values in respective groups treated with vehicle or drug. The M.E.D. (minimally effective dose) for a given compound is the lowest dose for which the mean value of this ratio is significantly lower than that obtained for the vehicle-treated group. The effect of the drugs in these assays can be partially blocked by metergoline, a known serotonin antagonist.

The compositions of the present invention may be formulated in a conventional manner using one or more pharmaceutically acceptable carriers. Thus, the active compounds of the invention may be formulated for oral, buccal, intranasal, parenteral (e.g., intravenous, intramuscular or subcutaneous) or rectal administration or in a form suitable for administration by inhalation or insufflation.

For oral administration, the pharmaceutical compositions may take the form of, for example, tablets or capsules prepared by conventional means with pharmaceutically acceptable excipients such as binding agents (e.g. pregelatinised maize starch, polyvinylpyrrolidone or hydroxypropyl methylcellulose) ; fillers (e.g. lactose, microcrystalline cellulose or calcium phosphate); lubricants (e.g. magnesium stearate, talc or silica); disintegrants (e.g. potato starch or sodium starch glycollate); or wetting agents (e.g. sodium lauryl sulphate). The tablets may be coated by methods well known in the art. Liquid preparations for oral administration may take the form of, for example, solutions, syrups or suspensions, or they may be presented as a dry product for constitution with water or other suitable vehicle before use. Such liquid preparations may be prepared by conventional means with pharmaceutically acceptable additives such as suspending agents (e.g. sorbitol syrup, methyl cellulose or hydrogenated edible fats); emulsifying agents (e.g. lecithin or acacia); non-aqueous vehicles (e.g. almond oil, oily esters or ethyl alcohol); and preservatives (e.g. methyl or propyl p-hydroxybenzoates or sorbic acid).

For buccal administration the composition may take the form of tablets or lozenges formulated in conventional manner.

The active compounds of the invention may be formulated for parenteral administration by injection, including using conventional catheterization techniques or infusion. Formulations for injection may be presented in unit dosage form e.g. in ampules or in multi-dose containers, with an added preservative. The compositions may take such forms as suspensions, solutions or emulsions in oily or aqueous vehicles, and may contain formulating agents such as suspending, stabilizing and/or dispersing agents. Alternatively, the active ingredient may be in powder form for reconstitution with a suitable vehicle, e.g. sterile pyrogen-free water, before use.

The active compounds of the invention may also be formulated in rectal compositions such as suppositories or retention enemas, e.g., containing conventional suppository bases such as cocoa butter or other glycerides.

For intranasal administration or administration by inhalation, the active compounds of the invention are conveniently delivered in the form of a solution or suspension from a pump spray container that is squeezed or pumped by the patient or as an aerosol spray presentation from a pressurized container or a nebulizer, with the use of a suitable propellant, e.g. dichlorodifluoromethane, trichlorofluoromethane, dichlorotetrafluoroethane, carbon dioxide or other suitable gas. In the case of a pressurized aerosol, the dosage unit may be determined by providing a valve to deliver a metered amount. The pressurized container or nebulizer may contain a solution or suspension of the active compound. Capsules and cartriges (made, for example, from gelatin) for use in an inhaler or insufflator may be formulated containing a powder mix of a compound of the invention and a suitable powder base such as lactose or starch.

A proposed dose of the compound (R)-5-(methylaminosulfonylmethyl)-3-(N-methylpyrrolidin-2-ylmethyl)-1H-indole for oral, parenteral or buccal administration to the average adult human for the treatment of the conditions referred to above (e.g., migraine) is 0.1 µg to 0.09 mg of the active ingredient per unit dose which could be administered, for example, 1 to 4 times per day. In another embodiment, the pharmaceutical composition includes 0.5 µg to 0.09 mg of the active ingredient per unit dose.

A proposed dose of the compound (R)-5-(methylaminosulfoylmethyl)-3-(pyrrolidin-2-ylmethyl)-1H-indole for oral, parenteral or buccal administration to the average adult human for the treatment of the conditions referred to above (e.g., migraine) is 0.01 µg to 0.09 mg of the active ingredient per unit dose which could be administered, for example, 1 to 4 times per day. In another embodiment, the pharmaceutical composition includes 0.05 µg to 0.09 mg of the active ingredient per unit dose.

Aerosol formulations for treatment of the conditions referred to above (e.g., migraine) in the average adult human are preferably arranged so that each metered dose or "puff" of aerosol contains 0.01 µg to 19 µg of either of the compounds (R)-5-(methylaminosulfonylmethyl)-3-(N-methyl-pyrrolidin-2-ylmethyl)-1H-indole or (R)-5-(methylaminosulfonylmethyl)-3-(pyrrolidin-2-ylmethyl)-1H-indole. In another embodiment, each metered dose or "puff" of aerosol contains 0.05 µg to 19µg of the active ingredient. The overall daily dose with an aerosol will be within the range 0.05 µg to less than 100 µg. In one embodiment, the overall daily dose with an aerosol will be within the range 0.05 µg to 99 µg of the active ingredient. Administration may be several times daily, for example 2, 3, 4 or 8 times, giving for example, 1, 2 or 3 doses each time.

The following Examples illustrate the preparation of the compounds of the present invention. Melting points are uncorrected. NMR data are reported in parts per million (δ) and are referenced to the deuterium lock signal from the sample solvent. Specific rotations were measured at room temperature using the sodium D line (589 nm).

Commercial reagents were utilized without further purification. Chromatography refers to column chromatography performed using 32-63 µm silica gel and executed under nitrogen pressure (flash chromatography) conditions. Room temperature refers to 20 - 25°C.

### EXAMPLE 1

### (R)-5-(Methylaminosulfonylmethyl)-3-(N-methyl-pyrrolidin-2-ylmethyl)-1H-indole

To a stirred mixture of lithium aluminium hydride (0.221 g, 5.82 mmol, 2 eq) in anhydrous tetrahydrofuran (15 mL) at O°C was added rapidly a solution of (R)-3-(N-benzyloxycarbonylpyrrolidin-2-ylmethyl)-5-(methylaminosulfonylmethyl)-1H-indole (2.97 mmol) in anhydrous tetrahydrofuran (5 mL). The resulting mixture was heated at reflux under a nitrogen atmosphere for 3 hours. The reaction mixture was cooled, and sodium sulfate decahydrate (5g) and water (0.5 mL) were added. The resulting mixture was stirred at 25°C for 8 hours, filtered, and the filtrate was evaporated under reduced pressure. The residue was column chromatographed using silica gel (approximately 50 g) and elution with a solution methylene chloride: methanol: ammonium hydroxide [9:1:0.1] to afford the title compound as a white solid (340 mg, 78%): mp, 213.0-214.0°C; ¹H NMR (DMSO-d₆) δ 10.9 (br s, indole NH), 7.51 (br d, 1H), 7.31 (d, J=8.3 Hz, 1H), 7.16 (br d, 1H), 7.08 (br dd, J=8.3 Hz, 1H), 6.82 (br q, sulfonamide NH), 4.35 (s, 2H), 3.07-2.95 (m, 2H), 2.54 (d, J=4.7 Hz, 3H), 2.52-2.38 (m, 2H), 2.35 (s, 3H), 2.10 (br, q, J=8.2 Hz, 1H), 1.75-1.40 (m, 4H); [α]²⁵=+89° (DMSO-d₆, c=1.0); Anal. calcd for C₁₆H₂₃N₃SO₂: C, 59.79; H, 7.21; N, 13.07. Found: C, 59.66; H, 7.29; N, 12.81.

### EXAMPLE 2

### (R)-5-(Methylaminosulfonylmethyl)-3-(pyrrolidin-2-ylmethyl)-1H-indole

A mixture of (R)-3-(N-Benzyloxycarbonylpyrrolidin-2-ylmethyl)-5-(methylaminosulfonylmethyl)-1H-indole (0.62 g, 1.40 mmol) and 20% Pd(OH)₂ on carbon (0.63 g) in absolute ethanol was shaken under a hydrogen atmosphere (3 atm) for 16 hours. The resulting reaction mixture was filtered through diatomaceous earth, and the filtrate was evaporated under reduced pressure. The residue was column chromatographed using silica gel (approximately 50 g) and elution with a solution of methylene chloride: methanol: ammonium hydroxide [8:2:0.2] to afford the title compound (0.216 g, 44%) as an off-white gum: ¹³C NMR (DMSO-d₆) δ 135.9, 127.5, 123.8, 123.7, 120.9, 119.7, 112.4, 111.1, 59.2, 56.6, 45.7, 31.1, 31.0, 29.0, 24.6; [α]²⁵ = +4° (DMSO-d₆, c=1.0); [α]²⁵=-14° (EtOH/CHCl₃ [1:1], c=1.0); HRMS: calculated for [C₁₅H₂₁N₃O₂S•H⁺]: 308.1433; found: 308.1467.

### EXAMPLE 3

### (R)-3-(N-Benzyloxycarbonylpyrrolidin-2-ylmethyl)-5-(methylaminosulfonylmethyl)-1H-indole

A mixture of (R)-1-(N-Benzyloxycarbonylpyrrolidin-2-yl)-3-(N-(2-bromo-4-methylaminosulfonylmethylphenyl)-N-trifluoroacetylamino)propene (4.00 g, 6.47 mmol), tetrabutylammonium chloride (1.84 g, 6.62 mmol), and palladium(II) acetate (.407 g, 1.82 mmol, 0.3 eq) in a solution of triethylamine (22 mL) and anhydrous N,N-dimethylformamide (5 mL) was heated at reflux under nitrogen for 1 hour. The resulting reaction mixture was evaporated under reduced pressure, and the residue was partitioned between ethyl acetate (100 mL) and water (100 mL). The ethyl acetate layer was removed, and the aqueous layer was extracted with additional ethyl acetate (100 mL). The organic extracts were combined, dried (MgSO₄), and evaporated under reduced pressure. The residue was column chromatographed using silica gel (approximately 400 g) and elution with an acetone gradient (0.5%-5%) in methylene chloride to afford the title compound (1.30 g, 45%) as an off-white foam: IR (CHCl₃) 1673, 1410, 1358, 1324, 1118, 1092 cm⁻¹; LRMS (m/z, relative intensity) 441 (9, M+), 237 (29), 204 (77), 160 (97), 143 (73), 91 (100); HRMS: calculated for C₂₃H₂₇N₃O₄S: 441.1724; found: 441.1704; [α]²⁵=-30° (CD₃OD, C=1).

### EXAMPLE 4

### (R)-1-(N-Benzyloxycarbonylpyrrolidin-2-yl)-3-(N-(2-bromo-4-methylaminosulfonylmethylphenyl)-N-trifluoroacetylamino)propene

To a stirred mixture of (R)-1-(N-benzyloxycarbonylpyrrolidin-2-yl)-3-hydroxypropene (3.75 g, 14.3 mmol), 2-bromo-4-methylaminosulfonylmethyl-N-trifluoroacetylaniline (4.45 g, 11.8 mmol) and triphenylphosphine (3.78 g, 14.4 mmol) in anhydrous tetrahydrofuran (60 mL) at 0°C under a nitrogen atmosphere was added diethyl azodicarboxylate (2.30 mL, 14.1 mmol) dropwise. The reaction solution was slowly warmed to 25°C over the course of 2 hours, and then stirred at 25°C under a nitrogen atmosphere for an additional 12 hours. The resulting reaction solution was evaporated under reduced pressure, and the residue was column chromatographed using silica gel (approximately 600 g) and elution with 4% acetone in methylene chloride afforded the title compound as a white foam (4.06 g, 46%): FAB LRMS (m/z, relative intensity) 620 ([MH+ with ⁸¹Br], 618 ([MH+ with ⁷⁹Br], 98), 576 (50), 574 (63), 512 (17), 484 (33); [α]²⁵=+18% (CH₃OH, C=1).

### EXAMPLE 5

### 2-Bromo-4-methylaminosulfonylmethyl-N-trifluoroacetylaniline

To a stirred solution of 2-Bromo-4-methylaminosulfonylmethylaniline (0.55 g, 2.00 mmol) and pyridine (0.18 mL, 2.22 mmol, 1.1 eq) in anhydrous methylene chloride (10 mL) at 0°C under a nitrogen atmosphere was added dropwise trifluoroacetic anhydride (0.31 mL, 2.19 mmol, 1.1 eq). The resultant reaction mixture was stirred at 0°C under a nitrogen atmosphere for 3 hours. A saturated solution of sodium hydrogen carbonate was added (15 mL), and this aqueous mixture was extracted with ethyl acetate (3 x 15 mL). The extracts were combined, dried (MgSO₄), and evaporated under reduced pressure. Evaporation of the ethyl acetate extracts afforded the title compound (0.675 g, 90%) directly as a white solid: mp, 164.0-166.0°C. Anal. calcd for C₁₀H₁₀BrF₃N₂O₃S: C, 32.02; H, 2.69; N, 7.47. Found: C, 32.18; H, 2.67; N, 7.30.

### EXAMPLE 6

### 2-Bromo-4-methylaminosulfonylmethylaniline

To a stirred solution of 4-Methylaminosulfonylmethylaniline (M.D. Dowle, et al. Eur. Pat. Appl. EP225,726) (0.40 g, 2.00 mmol) in methanol (10 mL) at 0°C was added dropwise bromine (0.113 mL, 2.19 mmol, 1.1 eq). The resulting reaction mixture was then stirred at 25°C for 30 minutes. The reaction mixture was then evaporated under reduced pressure, and the residue was placed in a saturated solution of sodium hydrogen carbonate (10 mL). This aqueous mixture was extracted with ethyl acetate (3 x 15 mL). The extracts were combined, dried (MgSO₄), and evaporated under reduced pressure. The residue was column chromatographed using silica gel (approximately 50 g) and elution with 40% ethyl acetate in hexanes afforded the title compound (0.145 g, 26%) as a white solid: mp, 104.0-107.0°C. Anal. calcd for C₈H₁₁BrN₂O₂S: C, 34.42; H, 3.97; N, 10.04. Found: C, 34.66; H, 3.96; N, 9.96.

### EXAMPLE 7

### (R)-1-(N-Benzyloxycarbonylpyrrolidin-2-yl)-3-hydroxypropene

To a stirred solution of (R)-ethyl 3-(N-benzyloxycarbonylpyrrolidin-2-yl)-2-propenoate (3.03 g, 10.00 mmol) in anhydrous tetrahydrofuran (75 mL) at -78°C under nitrogen was added dropwise a solution of diisobutylaluminium hydride (1.0 M in hexanes, 12.0 mL, 22.0 mmol, 2.2 eq). The resulting solution was stirred at -78°C under nitrogen for 30 minutes. The reaction solution was then allowed to warmed to room temperature over the course of 2 hours. A saturated solution of sodium hydrogen carbonate (50 mL) was added, and the aqueous mixture was extracted with ethyl acetate (3 x 50 mL). The extracts were combined, dried (MgSO₄), and evaporated under reduced pressure. Column chromatography of the residue with diethyl ether/hexanes [1:1] afforded the title compound (0.836 g, 32%) as a clear, colorless oil: ¹H NMR (CDCl₃) δ 7.40-7.25 (m, 5H), 5.75-5.53 (m, 2H), 5.20-5.00 (m, 2H), 4.38 (br m, 1H), 4.06 (br d, J=13.7 Hz, 2H), 3.45 (br t, J=7.0 Hz, 1H), 2.03-1.68 (m, 4H); [α]²⁵ = +34° (MeOH, c=1.0); HRMS: calculated for C₁₅H₁₉NO₃ 261.1365, found 261.1356.

### EXAMPLE 8

### (R)-Ethyl 3-(N-Benzyloxycarbonylpyrrolidin-2-yl)-2-propenoate

To a stirred solution of (R)-N-carbobenzyloxypyrrolidine-2-carboxaldehyde (1.17 g, 5.00 mmol) [S. Kiyooka, et al., J. Org. Chem., 5409 (1989) and Y. Hamada, et al., Chem. Pharm. Bull., 1921 (1982)] in anhydrous tetrahydrofuran at -78°C was added (carbethoxymethylene)triphenylphosphorane (2.09 g, 6.00 mmol. 1.2 eq) as a solid portionwise. The resulting reaction mixture was stirred at room temperature under nitrogen for 2 hours. The reaction mixture was evaporated under reduced pressure and the residue was column chromatographed using silica gel (approximately 100 g) and elution with 20% diethyl ether in hexanes to afford the title compound (1.26 g, 83%) as a clear, colorless oil: ¹H NMR (CDCl₃-d₆) δ 7.34-7.25 (m, 5H), 6.89-6.76 (m, 1H), 5.88-5.74 (m, 1H), 5.18-5.05 (m, 2H), 4.60-4.43 (m, 1H), 4.17 (q, J=7.1 Hz, 2H), 3.55-3.40 (m, 2H), 2.11-2.00 (m, 1H), 1.90-1.75 (m, 3H), 1.28 (t, J=7.1 Hz, 3H); ¹³C NMR (CDCl₃) [Note: due to slow nitrogen inversion two conformers of the products are seen by NMR spectroscopy] δ 166.3, 154.7, 147.9, 147.4, 136.6, 128.4, 127.9, 120.9, 66.9, 65.8, 60.4, 58.1, 57.7, 46.8, 46.4, 31.6, 30.8, 23.6, 22.8, 22.6, 15.3, 14.2; [α]²⁵=+61° (CH₃OH, C=1).

### EXAMPLE 9

### In vivo Assay of Plasma Protein Extravasation Response within The Dura Mater of Guinea Pigs

The procedure described previously in this application referencing Markowitz et al., J. Neurosci., 7 (12), 4129-4136 (1987) and in Lee, et al., Brain Reseach, 626, 303-305 (1993) was performed on (R)-5-(Methylaminosulfonylmethyl)-3-(N-methylpyrrolidin-2-ylmethyl)-1H-indole and (R)-5-(Methylaminosulfonylmethyl)-3-(pyrrolidin-2-ylmethyl)-1H-indole. The results for (R)-5-(Methylaminosulfonylmethyl)-3-(N-methylpyrrolidin-2-ylmethyl)-1H-indole was an ED₅₀=1.66 pmol/kg. The results for (R)-5-(Methylaminosulfonylmethyl)-3-(pyrrolidin-2-ylmethyl)-1H-indole was an ED₅₀=0.09 pmol/kg.

## Claims

1. A pharmaceutical composition for oral, parental buccal, or rectal administration comprising an amount of (R)-5-(methylaminosulfonylmethyl)-3-(N-methylpyrrolidin-2-ylmethyl)-1H-indole or a pharmaceutically acceptable salt thereof ranging from 0.1µg to 0.09 mg and a pharmaceutically acceptable carrier.

2. A pharmaceutical composition for oral, parental, buccal, or rectal administration comprising an amount of (R)-5-(methylaminosulfonylmethyl)-3-(pyrrolidin-2-ylmethyl)-1H-indole or a pharmaceutically acceptable salt thereof ranging from 0.01µg to 0.09 mg and a pharmaceutically acceptable carrier.

3. A composition as claimed in claim 1 wherein the amount ranges from 0.5µg to 0.09 mg.

4. A composition as claimed in claim 2 wherein the amount ranges from 0.5µg to 0.09 mg.

5. A pharmaceutical composition as claimed in any one of the preceding claims which is in the form of a tablet, capsule, suppository, retention enema, or unit dose for injection.

6. A pharmaceutical composition for aerosol administration comprising an amount of (R)-5-(methylaminosulfonylmethyl)-3-(N-methylpyrrolidin-2-ylmethyl)-1H-indole or (R)-5-(methylaminosulfonylmethyl)-3-(pyrrolidin-2-ylmethyl)-1-H-indole, or a pharmaceutically acceptable salt thereof, ranging from 0.01µg to 19µg per metered dose and a pharmaceutically acceptable carrier.

7. A composition as claimed in claim 6 wherein the amount ranges from 0.05µg to 19µg.

8. The use of a composition as claimed in any one of the preceding claims for the manufacture of a medicament for treating a condition selected from hypertension, depression, anxiety, eating disorders, obesity, drug abuse, cluster headache, migraine, pain and chronic paroxysmal hemicrania and headache associated with vascular disorders.

9. The use of a composition as claimed in any one of claims 1 to 7 for the manufacture of a medicament for treating a disorder arising from deficient serotonergic neurotransmission.

## Patentansprüche

1. Pharmazeutische Zusammensetzung zur oralen, parenteralen, bukkalen oder rektalen Verabreichung, umfassend eine Menge an (R)-5-(Methylaminosulfonylmethyl)-3-(N-methylpyrrolidin-2-ylmethyl)-1H-indol oder einem pharmazeutisch verträglichen Salz davon im Bereich von 0,1 µg bis 0,09 mg und einen pharmazeutisch verträglichen Träger.

2. Pharmazeutische Zusammensetzung zur oralen, parenteralen, bukkalen oder rektalen Verabreichung, umfassend eine Menge an (R)-5-(Methylaminosulfonylmethyl)-3-(pyrrolidin-2-ylmethyl)-1H-indol oder einem pharmazeutisch verträglichen Salz davon im Bereich von 0,01 µg bis 0,09 mg und einen pharmazeutisch verträglichen Träger.

3. Zusammensetzung nach Anspruch 1, wobei die Menge im Bereich von 0,5 µg bis 0,09 mg liegt.

4. Zusammensetzung nach Anspruch 2, wobei die Menge im Bereich von 0,5 µg bis 0,09 mg liegt.

5. Pharmazeutische Zusammensetzung nach einem der vorangehenden Ansprüche, die in Form einer Tablette, einer Kapsel, eines Suppositoriums, eines Retentionsklistiers oder einer Dosierungseinheit zur Injektion vorliegt.

6. Pharmazeutische Zusammensetzung zur Aerosolverabreichung, umfassend eine Menge an (R)-5-(Methylaminosulfonylmethyl)-3-(N-methylpyrrolidin-2-ylmethyl)-1H-indol oder (R)-5-(Methylaminosulfonylmethyl)-3-(pyrrolidin-2-ylmethyl)-1H-indol oder einem pharmazeutisch verträglichen Salz davon im Bereich von 0,01 µg bis 19 µg pro abgemessene Dosis und einen pharmazeutisch verträglichen Träger.

7. Zusammensetzung nach Anspruch 6, wobei die Menge im Bereich von 0,05 µg bis 19 µg liegt.

8. Verwendung einer Zusammensetzung nach einem der vorangehenden Ansprüche zur Herstellung eines Arzneimittels zur Behandlung eines Zustands, ausgewählt aus Hypertension, Depression, Beklemmung, Essstörungen, Fettsucht, Drogenmissbrauch, Klusterkopfschmerz, Migräne, Schmerz und chronischem paroxysmalem halbseitigem Kopfschmerz und Kopfschmerz, verbunden mit vaskulären Störungen.

9. Verwendung einer Zusammensetzung nach einem der Ansprüche 1 bis 7 zur Herstellung eines Arzneimittels zur Behandlung einer Störung, die durch mangelhafte serotonerge Neutrotransmission entsteht.

## Revendications

1. Composition pharmaceutique pour l'administration orale, parentérale, buccale ou rectale, comprenant une quantité de (R)-5-(méthylaminosulfonylméthyl)-3-(N-méthylpyrrolidine-2-ylméthyl)-1H-indole ou d'un de ses sels pharmaceutiquement acceptables allant de 0,1 µg à 0,09 mg et un support pharmaceutiquement acceptable.

2. Composition pharmaceutique pour l'administration orale, parentérale, buccale ou rectale, comprenant une quantité de (R)-5-(méthylaminosulfonylméthyl)-3-(pyrrolidine-2-ylméthyl)-1H-indole ou d'un de ses sels pharmaceutiquement acceptables allant de 0,01 µg à 0,09 mg et un support pharmaceutiquement acceptable.

3. Composition suivant la revendication 1, dans laquelle la quantité va de 0,5 µg à 0,09 mg.

4. Composition suivant la revendication 2, dans laquelle la quantité va de 0,5 µg à 0,09 mg.

5. Composition pharmaceutique suivant l'une quelconque des revendications précédentes, qui est sous forme d'un comprimé, d'une capsule, d'un suppositoire, d'un lavement à garder ou d'une dose unitaire pour l'injection.

6. Composition pharmaceutique pour l'administration en aérosol, comprenant une quantité de (R)-5-(méthylaminosulfonylméthyl)-3-(N-méthylpyrrolidine-2-ylméthyl)-1H-indole ou (R)-5-(méthylaminosulfonylméthyl)-3-(pyrrolidine-2-ylméthyl)-1H-indole, ou d'un de ses sels pharmaceutiquement acceptables, comprise dans l'intervalle de 0,01 µg à 19 µg par dose mesurée, et un support pharmaceutiquement acceptable.

7. Composition suivant la revendication 6, dans laquelle la quantité est comprise dans l'intervalle de 0,05 µg à 19 µg.

8. Utilisation d'une composition suivant l'une quelconque des revendications précédentes pour la production d'un médicament destiné au traitement d'une affection choisie entre l'hypertension, la dépression, l'anxiété, des troubles de l'alimentation, l'obésité, la dépendance de médicaments, les céphalées vasculaires de Horton, la migraine, la douleur, l'hémicranie paroxystique chronique et les céphalées associées à des troubles vasculaires.

9. Utilisation d'une composition suivant l'une quelconque des revendications 1 à 7 pour la production d'un médicament destiné au traitement d'une affection provenant d'une neurotransmission sérotoninergique déficiente.
